(19) Europäisches Patentamt
European Patent Office
Office européen des brevets

(11) **EP 2 744 445 B1**

(12) **EUROPÄISCHE PATENTSCHRIFT**

(45) Veröffentlichungstag und Bekanntmachung des Hinweises auf die Patenterteilung:
**31.01.2018 Patentblatt 2018/05**

(21) Anmeldenummer: **12761541.7**

(22) Anmeldetag: **20.08.2012**

(51) Int Cl.:
*A61F 2/00* (2006.01)  *A61F 5/00* (2006.01)
*A61M 3/02* (2006.01)  *A61M 25/10* (2013.01)

(86) Internationale Anmeldenummer:
**PCT/EP2012/003535**

(87) Internationale Veröffentlichungsnummer:
**WO 2013/026565 (28.02.2013 Gazette 2013/09)**

(54) **TRANS-ANALER EINLAUFKATHETER ZUR INTERMITTIERENDEN AUSLÖSUNG EINER REFLEXKOORDINIERTEN DEFÄKATION**

TRANS-ANAL INFLOW CATHETER FOR INTERMITTENTLY TRIGGERING A REFLEX-COORDINATED DEFECATION

CATHÉTER DE LAVEMENT TRANSANAL POUR DÉCLENCHER PAR INTERMITTENCE UNE DÉFÉCATION RÉFLEXE COORDONNÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: 20.08.2011  DE 102011111225
21.11.2011  DE 102011118943
16.12.2011  DE 102011121202
22.03.2012  DE 102012005607
25.04.2012  DE 102012008361

(43) Veröffentlichungstag der Anmeldung:
**25.06.2014 Patentblatt 2014/26**

(73) Patentinhaber: **Advanced Medical Balloons GmbH 68753 Waghäusel (DE)**

(72) Erfinder: **GÖBEL, Fred 69259 Wilhelmsfeld (DE)**

(74) Vertreter: **Küchler, Stefan Patentanwalt Färberstrasse 20 90402 Nürnberg (DE)**

(56) Entgegenhaltungen:
WO-A1-2006/010556    WO-A1-2008/103788
DE-A1-102009 008 594  FR-A1- 2 480 127
US-A1- 2007 021 651   US-A1- 2007 213 661

EP 2 744 445 B1

**Beschreibung**

Die Erfindung richtet sich einerseits auf eine Vorrichtung zum trans-analen Einbringen eines Einlaufs in das Rektum bzw. Colon eines Patienten, umfassend einen aufblasbaren Ballon von durch Präformierung bei der Herstellung vorgegebener, taillierter, insbesondere hantel- oder sanduhrförmiger Gestalt, mit zwei endständigen Ballonabschnitten von größerem Radius und etwa sphärischer oder diskoider Gestalt, sowie einem dazwischen angeordneten, mittigen, verjüngten Ballonabschnitt von verringertem Radius, welcher trans-anal platziert wird, derart, dass der distal anschließende, radial erweiterte Ballonabschnitt intra-rektal platziert ist und der proximal anschließende, radial erweiterte Ballonabschnitt extra-korporal, sowie andererseits auf ein Verfahren (nicht beansprucht) zum Befüllen des Katheterballons einer derartigen Vorrichtung zum trans-analen Einbringen eines Einlaufs in das Rektum bzw. Colon eines Patienten mittels einer Befüllvorrichtung.

Katheter zur trans-analen Applikation eines Einlaufes in den Enddarm (Rektum) bzw. den Dickdarm (Colon) eines Patienten sind in verschiedenster Ausführungsform seit längerem bekannt (siehe z.B. WO 2008/103788 A1). Einlaufkatheter können als einfache Schlauchelemente ausgeführt sein, zum verbesserten Halt des Katheters im Rektum aber auch mit einem intrarektal platzierten Ballonelement versehen werden. Derartige Ballonelemente bieten neben einer ano-rektal ankernden Funktion auch einen gewissen Dichtungseffekt, der die durch die Irrigation eingebrachte Flüssigkeit im Darm hält. Das Dichtungsvermögen von Einlaufkathetern mit ausschließlicher intra-rektaler Ballonkomponente ist in vielen Fällen jedoch nicht ausreichend und muss vom Anwender durch fortwährende manuelle Lagekorrektur der dichtenden Ballonflächen auf dem Rektumboden bzw. Manipulation der Stellung des dem Ballon aufsitzenden Katheterschaftes hergestellt werden.

Ein wesentliches Risiko bei der Anwendung von handelsüblichen, trans-anal in den Darm eingeführten Kathetern besteht nach wie vor in der Perforation der Darmwandung bei unsachgemäßer Handhabung oder auch bei vorgeschädigten bzw. entsprechend geschwächten Darmwandstrukturen. Derartigen Perforationen des Darms kann bislang nur durch eine entsprechende Schulung und Sensibilisierung des Anwenders auf diese besondere Problematik vorgebeugt werden.

Im Verlauf der letzten zehn Jahre hat sich über die Anwendung konventioneller trans-analer Einläufe hinaus eine neuartige Einlauftechnologie etabliert, die durch intermittierende, relativ kleinvolumige intra-rektal eingebrachte Einläufe die reflexkoordinierte aktive Entleerung des Rektums und weiten Anteilen des sich an das Rektum anschließenden linksseitigen Colon auslöst. Durch die konsequente Entleerung dieser Darmabschnitte kann der Patient so, trotz bestehender Inkontinenzproblematik, in den Zustand einer sogenannten "Pseudo-Kontinenz" überführt werden. Das Verfahren der sogenannten trans-analen Irrigation (TAI) kann vom Patienten selbst durchgeführt werden.

Die Funktion des rektal instillierten Einlaufmediums besteht neben einer gewissen Mobilisierung von Stuhl vor allem in einer moderaten Dehnung der Wandanteile der Rektumampulle (terminales Rektum), die in der Folge zur Auslösung des physiologischen Stuhlentleerungsreflexes (Reflextriggerung) führt. Während das rektal platzierte Einlaufmedium in der Regel bereits nach wenigen Minuten eine derartige Reflextriggerung verursacht, kann die sich anschließende vollständige Entleerung des angewendeten Irrigationsvolumens aus dem Rektum bzw. Colon deutlich längere Zeit, bis hin zu 30 Minuten oder darüber hinaus erfordern. Der relativ hohe Zeitbedarf der TAI schränkt die Akzeptanz bei vielen Patienten ein, und schließt die Methode, obwohl beim Einzelnen in der Regel effizient anwendbar, häufig aus.

Problematisch ist zudem, dass viele Anwender nicht in der Lage sind, die Irrigationskatheter konventioneller Bauart manuell in eine ausreichend dichtende Lage zu bringen und zu halten.

Ferner kann es bei der Aufdehnung der Ballonkomponente herkömmlicher Katheter innerhalb des Rektums bereits bei dessen beginnender Füllung zu einer reflextriggernden Dehnung von Darmwandanteilen kommen, die unter anderem zu einer reflexartigen Öffnung des Anus, und so unter Umständen zum Herausgleiten des ankernden Ballons aus dem Rektum führt.

Im Markt verfügbare Vorrichtungen zur intermittierenden trans-analen Irrigation, wie z.B. das System Peristeen - Anal Irrigation, der Fa. Coloplast, Dänemark, sind darüber hinaus relativ aufwendig konstruiert, und oft nur bedingt für die Anwendung außerhalb des vertrauten häuslichen Bereichs geeignet.

Wünschenswert für die verbesserte Akzeptanz der trans-analen Irrigation beim Anwender wäre eine Kathetertechnik, die den Katheterschaft nach dessen Einführen ins Rektum ohne erforderliche fortwährende Lagekorrektur, in seiner trans-analen Position hält, und sowohl das potentiell traumatische Hineingleiten des Schaftkörpers in den Darm verhindert, als auch das verfrühte unerwünschte Herausgleiten des Katheters aus dem Anus zuverlässig vermeidet.

Einen besseren Komfort hinsichtlich der transanalen Platzierung des Katheters bieten in dieser Hinsicht Einlaufkatheter mit Doppelballonanordnung. Dem Katheterschaft sitzen hier, von einander auf dem Schaft beabstandet, zwei separate, in der Regel über ein einziges Füll-Lumen gleichzeitig befüllte Ballonelemente auf. Diese kommen auf beiden Seiten des Analkanals, also innen und außen, zu liegen bzw. nehmen die Strukturen des Anus zwischen sich auf. Die in der Regel elastische Ausführung des Ballonmaterials führt bei Aufdehnung der Ballonwandung unter Druck zu einer sphärischen Erweiterung der Ballonkörper und so zu einer gewissen axialen wirkenden Quetschung des zwischen den Ballonkörpern liegenden Anus. Die dislokationssichere Positionierung des Katheterschaftes ist bei derartigen Kathetern als relativ sicher anzunehmen. Problematisch sind die zum Teil hohen, zur elastischen Aufdehnung der Hülle erforderlichen Fülldrucke, die von Patienten als störendes oder auch schmerzhaftes Fremdkörpergefühl empfunden werden. Ferner kann die elastische Aufdehnung des intra-rektalen Ballons zu einer prall gefüllten Sphäre zur unmittelbaren Triggerung des Defäkationsreflexes führen, was wiederum bei simultaner rektaler Kontraktion und nachlassendem Schließmuskeltonus im ungünstigsten Fall zum Herausgleiten des Katheters und zur verfrühten Entleerung der Irrigationsflüssigkeit führt.

Die DE 10 2004 033 425 B4 beschreibt ein Verschlußsystem zur Versorgung rektaler bzw. analer Inkontinenz mit einer besonderen Ausführungsform für die Tamponade blutender Hämorrhoiden, wobei ein tailliertes Ballonelement dargestellt ist, welches jeweils endständig über einen intra-rektalen bzw. prä-analen Abschnitt verfügt. Die Ballonhülle ist dabei derart auf dem ballontragenden Schaftkörper platziert, dass sich das intra-rektale Ballonsegment bei Befüllung zum Rektumboden hin bewegt und dort auf die blutenden venösen Gefäße einen tamponierenden Druck ausübt. Entsprechend bewegt sich das prä-anale Ballonsegment zur äußeren Analöffnung hin. So stellt sich eine von beiden Seiten axial auf den Anus gerichtete Tamponadewirkung ein. Zusätzlich zur axialen Blutungstamponade dehnt sich der mittlere taillierte Abschnitt des Ballons radial zur Wandung des Analkanals hin aus. Er ist bevorzugt mit einem Durchmesser ausgeformt, der den Durchmesser des geöffneten Analkanals überschreitet. Die Spitze des Schaftkörpers ragt bei der beschrienen Vorrichtung zur Akutversorgung einer venösen ano-rektalen Blutung im Zustand der Anwendung frei und ungeschützt in den Darmraum, und stellt ein potentielles Verletzungsrisiko dar.

Die WO 2007/118621 A1 beschreibt ein ähnliches hantel- bzw. sanduhrförmiges Ballon-Verschlußsystem. Ihm liegt die Aufgabe zu Grunde, für intermittierende Zeiträume von wenigen Stunden bei chronisch ano-rektal inkontinenten Patienten eine Dichtung gegen unwillkürlich austretenden Stuhl zu gewährleisten. Bei dieser Vorrichtung zur Versorgung steht ebenfalls die von beiden Seiten axial zum Anus hin gerichtete Rollbewegung der endständigen Ballonsegmente im Vordergrund. Auch hier ragt die Spitze des den Ballon tragenden Schaftkörpers im trans-anal platzierten Zustand frei und potentiell traumatisierend in den Darmraum.

Beide Vorrichtungen beschreiben eine sanduhr- bzw. hantelförmige Ballongestalt, wobei der Anus im taillierten, gegenüber den endständigen Portionen des Ballons verjüngten Bereich aufgenommen wird. Durch eine derartige Ausformung des Ballons kann neben einer relativ guten Sicherung des Katheters vor Dislokation, wegen der allseitigen Dichtung der Ballontaille innerhalb des Analkanals auch eine, gegenüber konventionellen Doppelballons deutlich verbesserte Verschlussleistung erreicht werden. Auch eine Irritationen, Schmerzen oder einen Defäkationsreflex auslösende Aufdehnung der Ballonhülle kann bei beiden Vorrichtungen weitgehend vermieden werden, da die Ballonwandung vorzugsweise bereits auf ihr Arbeitsmaß oder darüber hinaus ausgeformt ist. Zur Befüllung bzw. Entfaltung des Ballons in seinen Arbeitszustand ist daher lediglich ein niedriger Fülldruck erforderlich, der dem jeweils im Rektum bzw. im Bauchraum herrschenden Druck entspricht, oder diesen nur gering überschreitet.

Als besonders nachteilig bei den in DE 10 2004 033 425 B4 und WO 2007/118621 A1 beschriebenen Ausführungen erweist sich jedoch die sich bei Befüllung des Katheterballons einstellende frei exponierte distale Spitze des den Ballon tragenden Schaftkörpers, welche in das Darmlumen hineinragt und dort im Rahmen der Anwendung zu entsprechenden Irritationen oder Verletzungen der Darmwandung führen kann.

Das die Erfindung initiierende Problem besteht darin, diese Nachteile des bekannten Standes der Technik zu vermeiden. Die Erfindung ist in den Ansprüchen definiert. Die Lösung dieses Problems gelingt bei einer gattungsgemäßen Anordnung dadurch, dass die beiden Ballonenden sich auf das Schaftmaß des den Ballon tragenden Katheterschaftes verjüngen und in einfach umgestülpter bzw. invertierter Weise auf der vorzugsweise äußeren Mantelfläche des Katheterschaftes derart fixiert sind, dass sich bei Befüllung des Ballons die beiden radial erweiterten Ballonabschnitte in entgegengesetzten, axialen Richtungen aufeinander zu bewegen, und wobei die beiden radial erweiterten Ballonabschnitte gegenüber dem verjüngten, mittigen Ballonanteil derart vergrößert sind, dass beim trans-anal platzierten Ballon die beiden radial erweiterten Ballonabschnitte im Laufe der Befüllung sich über den mittigen, verjüngten Ballonabschnitt stülpen und im Grenzfall einander unmittelbar berühren und dadurch ihre Relativbewegung begrenzen und das distale Ende des Katheterschaftes daran hindern, den Scheitelpunkt des intra-rektalen Ballonradius bei axial maximal ausgelenkter Lage des Schaftkörpers nach distal hin zu überschreiten.

Um schaftspitzenbedingten Läsionen weitestgehend vorzubeugen, beschreibt die Erfindung ein spezifisches, besonders vorteilhaftes Verhältnis der Länge des mittigen taillenartigen Ballonsegmentes zur beidseitigen Einstülpung (Inversionen) der Fixierungspunkte der Ballonschaftenden auf dem Katheterschaft. Dieses Verhältnis gewährleistet, dass die Spitze des Katheterschaftes sich im befüllten, trans-anal positionierten Zustand spontan in das intra-rektale Ballonsegment retrahiert und dort atraumatisch eingebettet ist. Die atraumatische Sicherung der Katheterspitze innerhalb des intra-rektalen Ballonsegmentes ist erfindungsgemäß auch dann noch gewährleistet, wenn es zu anwendungstypischen Auslenkungen der Schaftachse innerhalb des Analkanals kommt, wie sie sich leicht durch Zug oder Stoß an der katheterzuleitenden Schlauchverbindung einstellen können.

Die Erfindung geht ferner auf das Problem der Vermeidung unerwünschter bzw. verfrühter Triggerwirkungen durch den befüllten, sich intra-rektal entfaltenden Ballon ein. Die Auslösung des Defäkationsreflexes kann in der Mehrzahl der Fälle durch eine Vorformung des Ballons auf Arbeitsmaß oder auch darüber hinaus (residuale Bemaßung) vermieden werden, da die zur Verankerung und Dichtung des Katheters erforderlichen Fülldrucke den im Rektum bzw. im Bauchraum herrschenden Drucken weitgehend entsprechen bzw. nur wenige Millibar darüber liegen müssen. Die atraumatische Sicherung der Katheterspitze im Bereich des intra-rektalen Ballonsegmentes ist erfindungsgemäß bereits bei derartig geringen Fülldrucken von beispielsweise 10 bis 25 mbar gewährleistet.

Die Ausformung des Ballons auf sein Arbeitsmaß erlaubt es dem Anwender ferner, durch die Erhöhung des Fülldrucks bzw. Füllvolumens, vom initialen Füllzustand primär dichtend und ankernd wirkenden Ballons, eine gut kontrollierbare, graduell steigerbare Dehnung der dem Katheterballon anliegenden Darmwandung herbeizuführen, die schließlich, bei individuell angepasster Intensität, zur kontrollierten Auslösung des Defäkationsreflexes führt.

Neben der initialen Dehnung der Wandung des Rektums kommt es bei entsprechender weiterer Steigerung des Fülldrucks im hantelförmig taillierten Katheterballon zudem zu einer Dehnung des Anus bzw. des analen Schließmuskels, was einen weiteren effizienten Triggerstimulus darstellt.

Die Intensität einer derartigen pneumatischen Dehnung der Darmwandung bzw. des Anus kann die Intensität einer reflex-auslösenden Dehnung mit flüssigen Medien deutlich überschreiten. Auf Grund des intensiveren Stimulus kann es trotz einer nur einmalig erfolgten Stimulation zur Auslösung von mehreren sequentiell ablaufenden Entleerungsreflexzyklen kommen.

Durch den vom Anwender gut steuerbaren pneumatischen Dehnungsstimulus kann zudem in vielen Fällen die Menge der erforderlichen Einlaufflüssigkeit reduziert werden, was wiederum die für die Entleerung des Einlaufs aus dem Rektum erforderliche Zeit erheblich verkürzen kann, und das Verfahren für viele Anwender so schließlich als Therapieoption für die Selbstanwendung erschließt.

Auf eine aufwendige technische Ausführung von Irrigationsgeräten kann bei der hier dargestellten optionalen pneumatisch-flüssig kombinierten Reflextriggerung verzichtet werden, da im bevorzugten Anwendungsfall das erforderliche Irrigationsvolumen so klein gehalten werden kann, dass es als kompakt anwendungsfertig zubereitete Lösung direkt mit einem erfindungsgemäß ausgeführten Einlaufkatheter über eine feste Zuleitung verbunden sein kann, und so in idealer Weise als Einmalprodukt zur Anwendung kommen kann.

Die irrigierende Lösung ist hierzu vorzugsweise in einem zylindrischen, beutelartigen Behälter abgefüllt, der vom Anwender bequem durch Auspressen mit der Hand intra-rektal eingebracht werden kann.

Der erfindungsgemäße Einlaufkatheter verfügt desweiteren über eine bevorzugt fest eingeklebte Füllleitung zur Beaufschlagung des Katheterballons mit Fülldruck. Die Einheit zur Befüllung des Ballons ist bevorzugt in wiederverwendbarer Form ausgeführt, und kann als einfacher, über eine Kupplung anschließbarer Handpumpballon oder auch Pumpballon mit druckanzeigendem Manometer gestaltet sein. Alternativ ist auch eine volumen-kontrollierte Befüllung des Ballons denkbar. Unabhängig von der Art der Befüllung, kann der Anwender durch schrittweise Näherung, die für sich optimale Ballonfüllung ermitteln, welche seiner individuellen Anatomie und seinem jeweiligen Reflexstatus bestmöglich angepasst ist.

Zur Gewährleistung einer möglichst praktikablen Kathetereinführung sowie einer sicheren trans-analen Platzierung des verjüngten Teils des hantel-förmigen Ballonelementes ist der Katheterschaft im proximalen, prä-analen Bereich vorzugsweise mit Griffmulden zur Aufnahme der den Katheter beim Einführen greifenden Finger ausgestattet. Falls der Anwender über keine Sensibilität im Becken- bzw. Analbereich verfügt, führt er den Katheter mit den Fingern bis zum Anschlag an den Anus ein und kann so ein unkontrolliert tiefes Einführen vermeiden.

Bevorzugt sind die Griffmulden auf dem Schaft im direkten proximalen Anschluss an die hintere Ballonfixierungslinie angebracht.

Die Erfindung zeichnet sich weiterhin aus durch ein den Ballonfülldruck anzeigendes Manometer. Dieses kann eine Skala aufweisen, auf welcher die für die Anwendung des erfindungsgemäßen Katheters erforderlichen Fülldruckbereiche durch entsprechende Markierungen vorgegeben sind.

Bevorzugt zeichnet sich ein erfindungsgemäßes Verfahren dadurch aus, dass durch die sequentielle pneumatische Triggerung eines Defäkationsreflexes eine das Volumen der Irrigationsflüssigkeit auf ein Maß von ca. 80 bis 120 ml pro Anwendung reduziert wird, wobei die Flüssigkeit bevorzugt als anwendungsfertige Lösung durch eine bevorzugt feste Zuleitung zu dem erfindungsgemäßen Katheter als Produkt zur Einmalanwendung zugeführt wird.

Weitere Merkmale, Eigenschaften, Vorteile und Wirkungen der Erfindung ergeben sich aus der folgenden Beschreibung bevorzugter Ausführungsformen der Erfindung sowie anhand der Zeichnung. Hierbei zeigt:

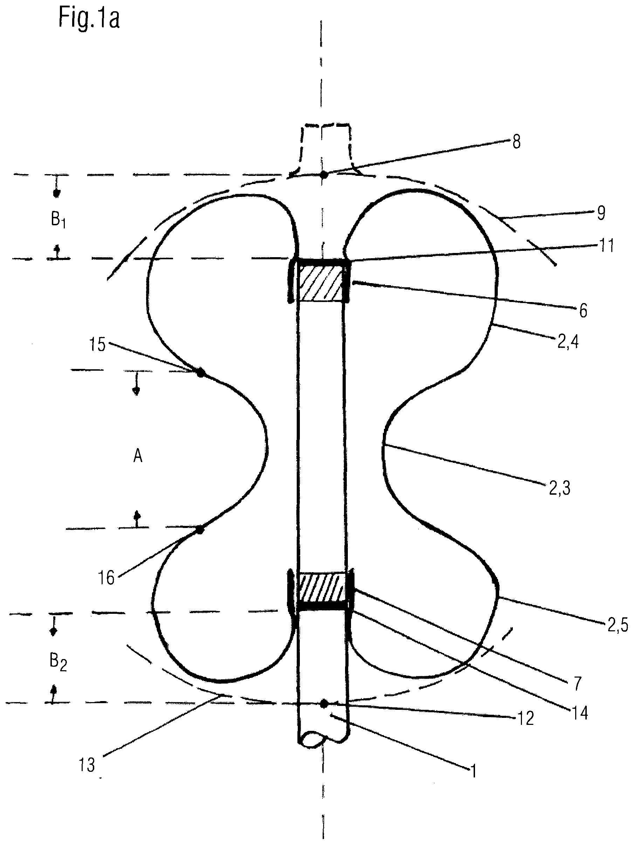

Fig. 1a     eine Ausführungsform eines erfindungsgemäßen Katheters in einem schematischen Längsschnitt, wobei die Ballonenden in erfindungsgemäß invertierter Weise auf dem Katheterschaft aufgebracht sind;

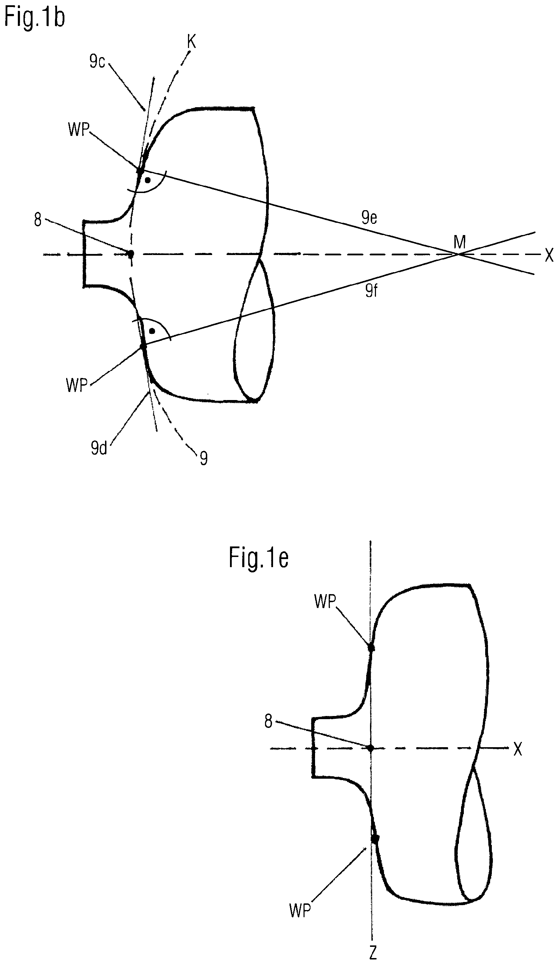

Fig. 1b     die geometrische Konstruktion des Scheitelpunktes des distalen Ballonradius;

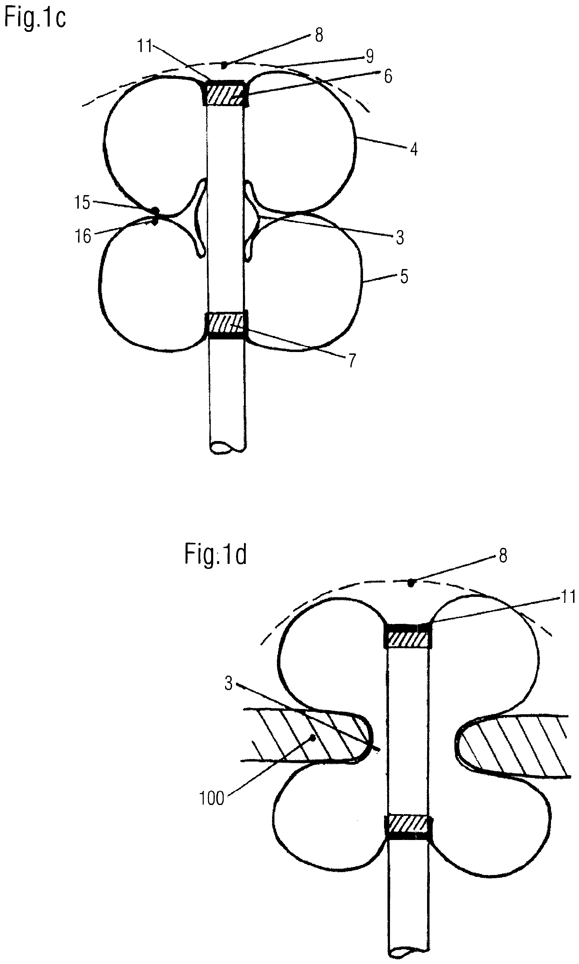

Fig.1c     den in Fig.1a beschriebenen Ballonkörper in frei entfaltetem, mit geringem Fülldruck beaufschlagten Zustand, außerhalb des Anus;

Fig.1 d    den in Fig.1a beschriebenen Ballonkörper in mit Fülldruck beaufschlagtem, trans-anal platzierten Zustand;

Fig. 1e    die geometrische Konstruktion der distalen Tangentialebene an den intrarektalen Ballonabschnitt;

Fig. 2    eine der Fig. 1a entsprechende Darstellung einer anderen Ausführungsform der Erfindung, wobei eine Katheterspitze über die vordere Fixierungslinie des Ballonschaftendes hinaus reicht;

Fig. 3    eine der Fig. 2 entsprechende Darstellung, wobei weitere Hilfslinien eingezeichnet sind, derart, dass der Bezugspunkt für die Bestimmung der Inversionstiefe B nicht der vordere Ballonradius ist, sondern der größte Durchmesser D des intra-rektalen Ballonsegmentes;

Fig. 4    eine ausgeformte Ballonhülle in nicht befülltem Zustand, die in besonders vorteilhafter Weise für das rektale Einführen und die sichere trans-anale Platzierung und Entfaltung des Ballons vorgesehen ist;

Fig. 5    eine abgewandelte Ausführungsform der Erfindung mit in ihrem transanalen Abschnitt tailliertem Katheterschaft;

Fig.6    ein manuell bedienbares Pumpmanometer mit einer für eine mehrstufige, sequentielle Befüllung des Katheterballons ausgestatteten Druckskala; sowie

Fig. 7    eine wiederum abgewandelte Ausführungsform der Erfindung mit fest mit dem Katheterschaft verbundenem Einlaufbehälter und Füllschlauch zur Beaufschlagung des Katheterballons mit Fülldruck.

Fig. 1a zeigt einen Längsschnitt durch einen Einlaufkatheter mit einem distal auf dem Katheterschaft 1 endständig aufgebrachten hantel- oder sanduhrförmigen Ballonelement 2. Das Ballonelement weist jeweils an den Enden beispielsweise sphärische oder diskusartige Erweiterungen auf. Im mittigen Bereich zwischen den endständigen Erweiterungen befindet sich ein im Durchmesser reduziertes, zylindrisch oder annähernd zylindrisch ausgeformtes Segment 3, welches die intrarektale Erweiterung 4 mit der prä-analen Erweiterung 5 durchgängig verbindet.
Der Ballon 2 weist endständig zwei Ballon-Schaftenden (6,7) zur Befestigung des Ballons auf dem Schaft auf. Die Schaftenden 6 und 7 werden bei der Ballonmontage beidseitig um einen bestimmten Betrag B in das Balloninnere eingestülpt (invertiert) und in dieser, zu einander hin versetzten Position, auf dem Schaft 1 durch beispielsweise Klebung oder Verschweißung fixiert.
Die Summe der Beträge B der beidseitigen Inversion soll dabei mindestens der Länge des verjüngten Zwischenstückes A entsprechen (A kleiner/gleich Summe der Beträge B).
Die Inversionstiefe B auf der dem Patienten bzw. dem Rektum zugewandten Ballonseite, entspricht der Distanz des Scheitelpunktes 8 des distalen, intrarektalen Ballonradius 9 zur distalen Fixierungslinie 11 des Ballonendes 6 auf dem Katheterschaft.
Der distale Radius 9 entspricht dem frontalen Radius bei freier, nicht invertierter Entfaltung des vollständig befüllten, jedoch nicht mit Druck beaufschlagten Ballons (gestrichelte Linie). Eine beispielhafte Vorschrift zur geometrischen Ermittlung des Scheitelpunktes 8 mit guter Näherung, ist in Fig. 1b dargestellt. Man erkennt insbesondere die beiden Wendepunkte WP des Längsschnitt durch das distale Ballonende, woraus durch Inversion des distalen Ballonbereichs der sphärische oder diskoide oder etwa halb-toroidale Bereich des intra-rektalen Ballonabschnitts resultiert. Die Lotrechte 9e, 9f an die Tangente an den Ballon-Längsschnitt schneiden die Längsachse X des Kathetersschaftes in dem Punkt M, und ein Kreis K um diesen Punkt M mit dem Abstand M-WP liefert den vorderen Scheitelpunkt 8, der die distale Grenze für den Katheterschaft in dessen neutraler, nicht ausgelenkter Ausgangsposition markiert.
In Fig. 1e ist zu sehen, dass man durch die Punkte WP auch eine Gerade legen kann, welche die Ebene Z repräsentiert, die nach Umstülpung des vorderen Ballonendes gerade eben den intra-rektalen Ballonabschnitt von distal tangiet und ebenfalls als Maß für die distalste Position des Katheterschaftes in dessen neutraler, nicht ausgelenkter Ausgangsposition angesehen werden kann.
Auf der dem Patienten abgewandten Ballonseite entspricht die Inversionstiefe B der Distanz des Scheitelpunktes 12 des proximalen, prä-analen Ballonradius 13 zur proximalen Fixierungslinie 14 des Ballonendes 7 auf dem Katheterschaft.
Der Radius 13 entspricht dem proximalen Radius bei freier, nicht invertierter Entfaltung des Ballons. Die geometrische Ermittlung des Scheitelpunktes 12 entspricht der in Fig. 1b beschriebenen Näherung.
Die Bestimmung der Länge des Zwischenstückes A erfolgt durch die Bestimmung der Distanz zwischen den Übergängen der Schulterradien 15 und 16 (Wendepunkte) der einander zugewandten Schulterflächen der Ballonsegmente 4 und 5.
Die Bestimmung der Inversionstiefen, Längen und Distanzen erfolgt jeweils im gefüllten, mit Fehldruck beaufschlagten Zustand, wobei der Fülldruck so gewählt wird, dass sich der Ballon vollständig entfaltet, sich jedoch keine elastische Aufdehnung der Ballonhülle einstellt.

Die Inversionstiefe B berechnet sich wie folgt: B >= A/2 (>= entspricht: größer/gleich).

Bei der Montage des Ballons auf dem Katheterschaft ist der Bezugspunkt für die jeweilige Inversion der Ballonschaftenden zum einen der Scheitelpunkt 8 des distalen, intra-rektalen Ballonradius 9, zum anderen der Scheitelpunkt12 des proximalen, prä-analen Ballonradius 13.

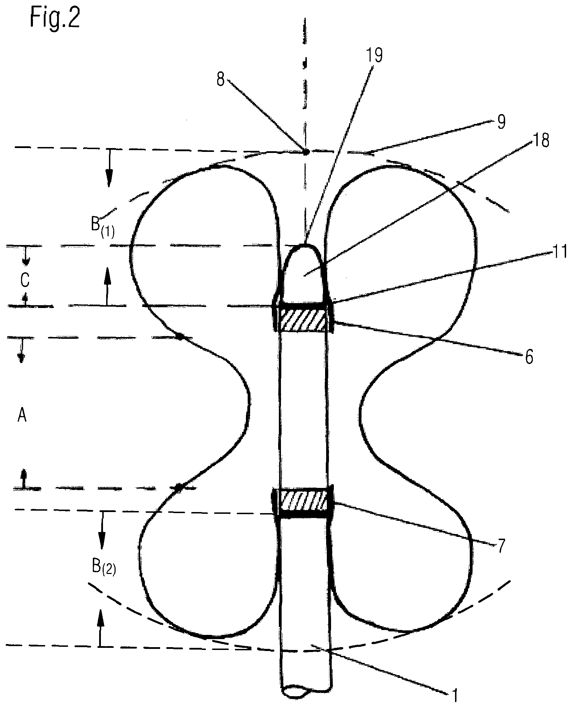

Bei der in dieser Abbildung beschriebenen Ausführungsform entspricht die distale Fixierungsline 11 auch dem distalen Ende des Katheterschaftes 1. Der Schaft schließt direkt mit der Fixierungslinie 11 ab und reicht nicht, wie in Abbildung 2 dargestellt, über diese Fixierungslinie nach distal hinaus.

Fig. 1b beschreibt die geometrische Herleitung des Scheitelpunktes des distalen Ballonradius.

Der vordere, frontal zum Darmlumen hin weisende Ballonradius 9 des intra-rektalen Ballonsegmentes 4 ist als gestrichelte Linie dargestellt. Er wird aus den beiden Wendepunkten 9a und 9b sowie den beiden, diesen Punkten jeweils zugehörigen Wendetangenten 9c und 9d konstruiert.

Mit Hilfe einer der beiden Wendepunkte 9a oder 9b wird eine Gerade 9e oder 9f konstruiert, welche zum einen lotrecht zur jeweiligen Wendetangente 9c oder 9d ist und den entsprechenden Wendepunkt 9a oder 9b schneidet. Der Schnittpunkt dieser Geraden 9e oder 9f mit der Symmetrieachse X ergibt den Mittelpunkt des Kreises K.

Der Kreis K, und damit der vordere Ballonradius 9 ergibt sich aus dem Kreismittelpunt M und den Wendepunkten 9a und 9b, welche sich auf dem Kreisumfang befinden. Der Scheitelpunkt 8 ergibt sich bei dieser Herleitung aus dem Schnittpunkt des Kreisumfangs mit der Symmetrieachse X des Ballons.

Für die im Folgenden verwendete Beschreibung des frontalen Scheitelpunktes 8 wird zur vereinfachten Herleitung des jeweils am weitesten nach distal reichenden Punktes der befüllten, nicht mit Druck beaufschlagten Ballonhülle der Schnittpunkt der Verbindungslinie Z der beiden Wendepunkte 9a und 9b mit der Symmetrieachse X verwendet.

Fig. 1c zeigt, wie sich der in Fig. 1a erfindungsgemäß invertierte Katheterballon bei freier Entfaltung und ohne Beaufschlagung mit Druck, im freien, nicht trans-anal platzierten Zustand verhält. Gezeigt wird die durch die spezifische Inversion der Ballonenden 6 und 7 auf dem Katheterschaft ermöglichte Gegenrollbewegung der beiden endständigen Ballonsegmente 4 und 5.

Bei einer bevorzugt besonders dünnwandigen und weichfolienartigen Ausführung des Ballonkörpers bewegen sich die beiden Segmente bereits bei geringstem, nahezu ambientem Fülldruck aufeinander zu und überrollen das mittige Segment 3. Bei Kontakt der beiden Segmente im Bereich der Übergangspunkte (15,16) der Schulterradien schließt der Scheitelpunkt 8 des Radius 9 bündig bzw. nahezu bündig mit der distalen Fixierungslinie 11 des distalen Ballonschaftendes 6 auf dem Katheterschaft ab.

Eine derartige Konfiguration würde in situ einer klinischen Anwendungssituation bei maximal verkürzter Länge des Analkanals entsprechen. Auch in diesem Extremfall wäre somit, bedingt durch die beschriebene Inversionsvorschrift gewährleistet, dass das freie distale Katheterschaftende, welches hier der distalen Fixierungslinie 11 entspricht, nicht in das Darmlumen hineinreicht und selbst bei maximaler seitlicher Auslenkung des Katheterschaftes im Rektum (Kippung des intra-rektalen Schaftanteils zur Darmwand) nicht mit der Wandung des Darms in Kontakt kommt und den distalen Ballonradius 9 als eine, Irritationen und Läsionen der Darmwand ausschließende, maximale Grenze überschreitet.

Fig.1d zeigt, wie sich die in Fig.1a beschriebene Invertierung der Ballonenden in Bezug zum distalen Katheterschaftende bei normaler oder nur geringfügig verkürzter Länge des Analkanals darstellt. Bei dieser, eher regulären klinischen Anwendungssituation stellt sich die vordere Fixierungslinie 11, welche hier wiederum dem distalen freien Ende des Katheterschaftes entspricht, deutlich in das Innere des intra-rektalen Ballons 4 versetzt dar. Die beiden endständigen Ballonsegmente 4 und 5 bewegen sich bei Beaufschlagung des Ballons mit Druck gegenläufig auf den Anus zu und schmiegen sich so dem jeweiligen analen Situs dichtend an. Bei entsprechend dünnwandiger Ausführung und weichfolienartigem Charakter der Ballonhülle stellt sich das Gegenrollen der Ballonsegmente ebenfalls bereits bei einer sehr geringen, zur Umgebung nahezu ambienten wirkenden Druckkraft (Fülldruck) ein.

Bereits der am trans-anal platzierten Ballon anliegende bzw. auf den Ballon wirkende individuelle, intra-abdominelle Druck führt zu einer kombinierten trans-analen Dichtwirkung aus radialer Dichtung gegen den Analkanal und einer von axial her gerichteten dichtenden Gegenrollbewegung der endständigen Ballonerweiterungen an den inneren und äußeren Ausgang des Anus. Die Dichtwirkung, ist daher nicht von einer initialen Befüllung des Ballons über sein Volumen bei freier Entfaltung hinaus, bis zur beginnenden Dehnung seiner Ballonhülle abhängig. Der Ballon kann sich auch bei partieller Befüllung, von beispielsweise 70 bis 90 % seines auf dem Katheterschaft frei entfalteten Volumens, in beschriebener axial gegenrollender und sich radial entfaltender Weise verhalten. Hierdurch ist eine nahezu druckneutrale und irritationsfreie Platzierung des Ballonkatheters im Anus möglich.

Eine traumatisierende Einwirkung der Katheterschaftspitze auf die Darmwandung kann somit bei normaler oder wenig veränderter analer Anatomie durch die sich erfindungsgemäß einstellende Inversion der Schaftspitze auch bei völlig druckneutraler Beaufschlagung mit einem Füllmedium ausgeschlossen werden.

Fig. 2 zeigt beispielhaft, wie Abschnitte des Katheterschaftes, welche die distale Fixierungslinie 11 in distaler, darmwärts gerichteter Fortführung als Spitzenstück 18 überschreiten, bei der Bestimmung der Inversionstiefe der Ballonenden 6 und 7 zu berücksichtigen sind, um eine erfindungsgemäßes atraumatische Inversion des distalen Katheterendes im intra-rektalen Ballon bei trans-analer Platzierung des befüllten Katheterballons zu gewährleisten. Die Länge C des Spitzenstückes 18 wird definiert als Distanz der vorderen Fixierungslinie 11 zum vorderen Scheitelpunkt 19 des Spitzenstückes.

Die Länge B verlängert sich im Vergleich zu Abb.1a um den Betrag der Länge C bzw. C/2.

Die korrespondierende Inversionstiefe B berechnet sich bei Berücksichtigung eines Spitzenstücks bevorzugt nach: B >= A/2 + C.

Alternativ hierzu kann die korrespondierende Inversionstiefe B bei Berücksichtigung eines Spitzenstücks weniger bevorzugt nach: B >= A/2 + C/2 erfolgen.

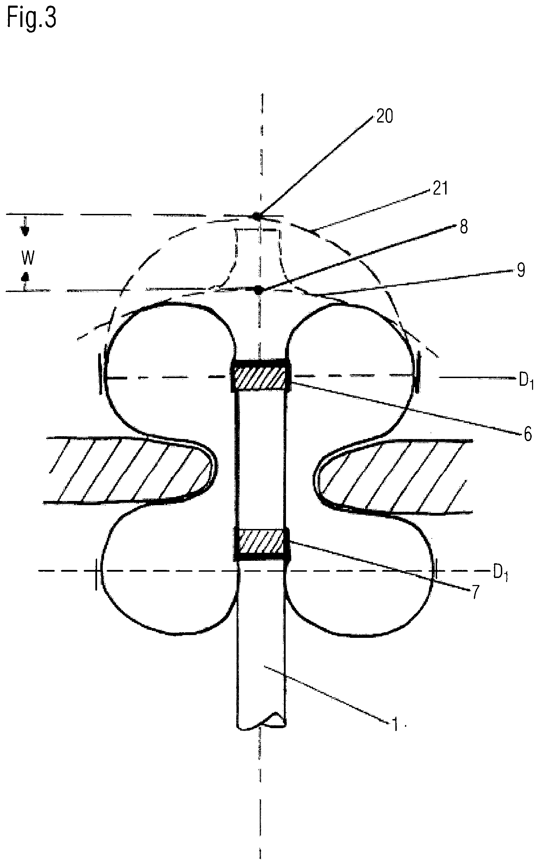

Fig. 3 stellt eine weitere, alternative Vorschrift zur Festlegung der Inversionstiefe B der Ballonschaftenden 6 und 7 auf dem Katheterschaft 1 in Bezug zum distalen Katheterschaftende 11, 19 dar.

Hierbei wird insbesondere eine mögliche axial gerichtete Verschiebung des Katheterschaftes innerhalb des befüllten, trans-anal platzierten Ballons berücksichtigt. Eine derartige Auslenkung des Schaftes in der Längsachse könnte im Rahmen der erfindungsgemäß beschriebenen Inversion der Ballonschaftenden auf dem den Ballon tragenden Katheterschaft zu darmwärts gerichteten Verschiebungen des distalen Katheterschaftende führen, und somit ein potentielles Perforationsrisiko darstellen.

Als maximaler distaler Auslenkungsweg W der vorderen Fixierungslinie 11 wird eine Distanz definiert, die sich als distal gerichtete Verlängerung der Schaftlängsachse vom Scheitelpunkt 8 des Radius 9 ausgeht und bis zum Scheitelpunkt 20 eines Radius 21 reicht, wobei der Radius 21 über dem größten Durchmesser D des intra-rektalen Ballonsegmentes 4 errichtet wird.

Verfügt der Katheterschaft über ein Spitzenstück 18, welches über die Linie 11 hinausreicht, soll der maximale Auslenkungsweg W entsprechend so gewählt werden, dass die Spitze 19 des Spitzenstücks bei maximaler Auslenkung W des Schaftes nicht über den Radius 21 reicht.

Der durch den größten Durchmesser D im intra-rektalen Ballonsegment definierte Radius 21 stellt eine prinzipiell relevante Begrenzungslinie für distale Katheterschaftanteile dar. Bei einer seitlichen Verkippung der Schaftlängsachse des trans-anal platzierten Katheterschaftes, ist durch den Bezug der maximalen Auslenkung W auf den größten Ballondurchmesser D gewährleistet, dass die Katheterschaftspitze (11,19) sich noch innerhalb des Schwenkradius 21 des Ballonsegmentes 4 bewegt, und somit ein potentiell traumatisierende Kontakt der Spitze mit der dem Ballon anliegenden Darmwand relativ gut ausgeschlossen werden kann.

Vorzugsweise soll bei der Bestimmung der Inversionstiefe B das jeweilige Verhältnis der Distanz W zum Radius 21 bzw. des ihm zugrundeliegenden Durchmessers D eingehalten und die Inversionstiefe B bei Bedarf entsprechend angepasst werden.

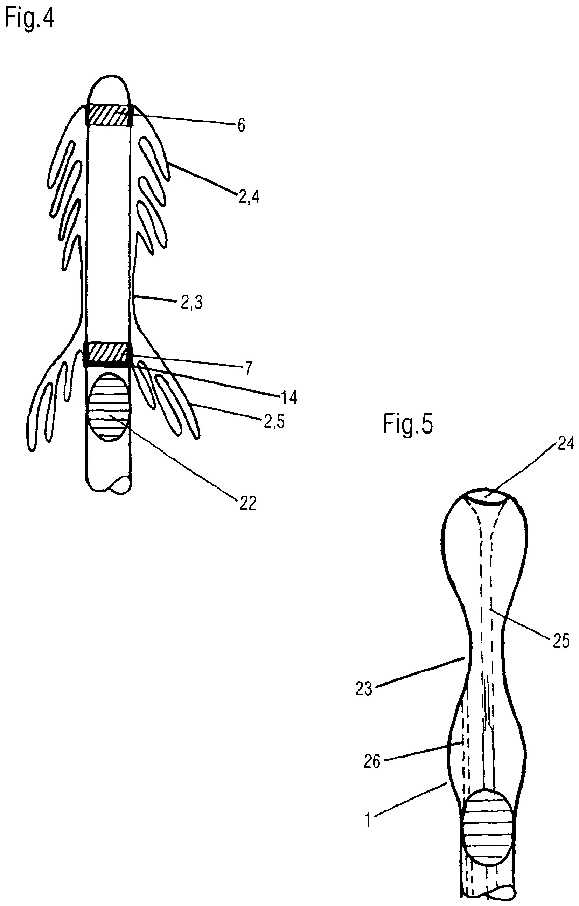

Fig. 4 zeigt den Katheterballon 2 in entleerter, dem Katheterschaft anliegender Form, wie er für die Einführung in den Anus bereitgestellt wird. Die Hüllensegmente des intra-rektalen Ballons 4 sowie des mittigen Segmentes 3 liegen dem Schaft in Faltung angeschmiegt an.

Dabei kommen die beiden Hüllenanteile vorzugsweise etwa auf Höhe der Strecke zwischen den Fixierungspunkten der Ballonenden 6 und 7 auf der Schaftoberfläche zu Liegen. Die Hülle des prä-analen Ballonsegmentes 5 wird hingegen bevorzugt nach proximal ausgestrichen und überragt die den Katheter beim Einführen greifenden Finger, wobei der bevorzugte Griffpunkt sich unmittelbar proximal von der proximalen Fixierungslinie 14 befindet. Der Griffpunkt 22 ist bevorzugt als muldenartige Aufnahmefläche ausgeführt, welche bevorzugt auf den beiden, um 180 Grad versetzten, gegenüberliegenden Schaftflächen aufgebracht sind.

Der Anwender greift bei einer derartigen Fixierung des evakuierten Ballons mit seinen Fingern unter die nach proximal ausgestrichene Hülle des Segmentes 5 und führt den Katheter bis zum Anschlag der greifenden Finger am äußeren Anus in das Rektum ein. Somit ist eine definierte Einführtiefe gewährleistet. Ferner ist sichergestellt, dass der intra-rektale Ballonanteil 4 in die rektale Kavität eingeführt ist, während der proximale Ballonanteil 5 außerhalb des Anus (prä-anal) zu liegen kommt. Bei der Befüllung des taillierten Ballons stellt sich somit eine sichere transanale Positionierung des Katheters ein.

Fig. 5 zeigt eine bevorzugte Ausführung des Schaftkörpers 1, die bereits eine gewisse selbsttätige Positionierung und Sicherung des Schaftes im Anus bietet, wenn der Katheterballon noch nicht befüllt ist. Der Schaft 1 weist hierzu eine ebenfalls taillierte Form auf, die im trans-analen Bereich 23 entsprechend verjüngt ist, und den Katheterschaft in diesem Bereich nach dem Einführen quasi in trans-analer Position einrastet. Der Katheterschaft weist bevorzugt zudem eine distal endständige, trichterartig mündende, atraumatisch ausgeformte Öffnung 24 auf, der sich der das Medium zuleitende Kanal 25 anschließt.

Bei einer besonders großvolumigen Ausführung des intra-rektalen Ballonsegmentes bzw. einer weit in das Rektum reichenden Längsausdehnung des Ballonsegmentes, kann dieses im entlüfteten, anwendungsfertigen Zustand optional partiell in die Öffnung 24 hineingestopft bzw. verpackt sein. Bei Befüllung des eingeführten Katheters schlüpft sie dann aus der Öffnung heraus.

Die Taille 23 kann zudem, bei angepasster Ausführung des Schaftmaterials eine gewisse Knickbarkeit des Schaftkörpers vorgeben, was dessen atraumatische Eigenschaften verbessert.

Die Befüllung des Ballons erfolgt durch einen separaten schaftintegrierten Kanal 26.

Zur Vermeidung des Rückstroms von Irrigationsflüssigkeit kann der Einlaufkatheter im Bereich des flüssigkeitsführenden Kanals 25 mit einem Rückschlagventil ausgestattet ist.

Das Ventil kann vorzugsweise aus einem dünnwandigen Schlauchelement von wenigen, vorzugsweise 5 bis 15 Mikrometern Wandstärke und dem Durchmesser des Kanals 25 bestehen, wobei das distale Ende des Schlauches über eine Länge von ca. 5 bis 10 mm frei im Kanal 25 liegt, und dessen proximales Ende dicht schließend mir der Innenwandung des Kanals 25 verbunden ist. Bei spitzenwärts gerichtetem Durchfluss des Mediums durch den Kanal öffnet sich das Schlauchelement und erlaubt den freien Durchfluss des Mediums. Bei entgegengesetzt gerichtetem Fluss kollabiert das Schlauchelement und verschließt sich dichtend, einen effektiven Rückstrom verhindernd.

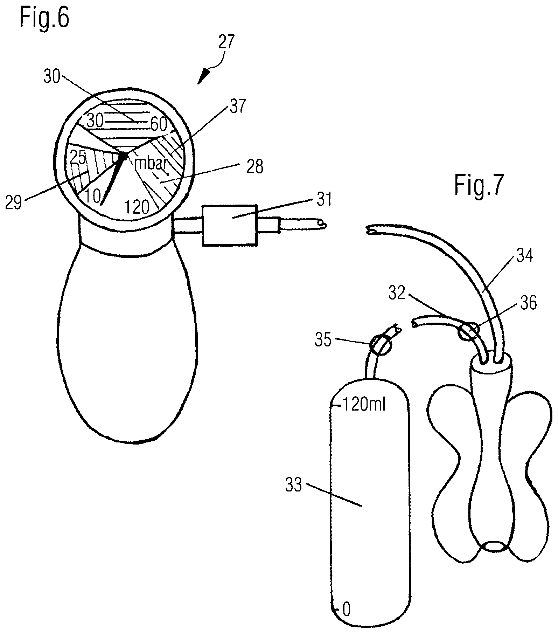

Fig. 6 zeigt schematisch ein Pumpmanometer 27 mit einer, zu einer mehrschrittigen bzw. sequentiell den Fülldruck steigernden Befüllung des erfindungsgemäßen Katheterballons geeigneten Skala 28. Die Skala stellt bevorzugt zum einen, einen initialen, niedrigen Druckbereich 29 (ca. 10-25 mbar) dar, der vom Anwender nach dem Einführen des Katheters und vor dem Einbringen der Einlaufflüssigkeit eingestellt wird, und bei erfindungsgemäßer Vorformung und Schaftfixierung der Ballonhülle in der Mehrzahl der Fälle bereits eine transanale Ankerung und Dichtung des Katheters gewährleistet, ohne einen unmittelbaren reflex-triggernden Effekt auf die Darmwand zu haben.

Bei einer durch den Anwender in Folge ausgelösten Steigerung des Fülldrucks im Ballon in den Bereich 30 (30-60 mbar, mit anzunehmender korrespondierender Dehnung rektaler Darmwandanteile) oder in den Bereich 37 (60-120 mbar, mit anzunehmender zusätzlicher Dehnung des analen Sphinkters) kann der Anwender schließlich einen in seiner Intensität weitgehend reproduzierbaren Triggerstimulus zur Auslösung eines Defäkationsreflexes erzeugen. Der Anwender hat somit den Vorzug, bei einem initial niedrigen, lediglich ankernd und dichtend wirkenden Ballondruck einen Entleerungsreflex weitgehend vermeiden zu können, und damit die Einlaufflüssigkeit für seinen individuellen Bedarf ausreichend lange im Darm halten zu können, was zu einer besseren Lösung bzw. Suspension des Stuhls in der Flüssigkeit führt. Andererseits kann er durch eine willkürlich herbeigeführte Druckerhöhung im Ballon einen intensiven, relativ prompt wirkenden, reflex-auslösenden Stimulus erzeugen, der hinsichtlich seiner Intensität, bei Bedarf, über die Triggerwirkung einer colo-rektalen Flüssigkeitssäule hinausgeht.

Die Befüllung des Katheterballons erfolgt bevorzugt durch Luft über eine in die Schaftwandung des Katheterkörpers 1 integrierte Füll-Leitung.

Neben einer, wie in Abbildung 6 dargestellten druckkontrollierten Befüllung des Ballons mit einem Pumpmanometer, kann zur volumen-kontrollierten Befüllung ein beigefügtes Spritzenelement verwendet werden, welches das bevorzugte Füllvolumen durch eine entsprechende Markierung auf dem Spritzenkörper vorgibt. Die Befüllung des Ballons erfolgt bei einer ein-schrittigen Befüllung bevorzugt partiell. Der Ballon liegt im idealen Fall also den Strukturen des Darms und des Anus in schlaffer, nicht-gedehnter Form an. Die Ballonhülle nimmt so die jeweilig im Rektum, im Anus und von präanal auf den Ballon einwirkenden Kräfte auf, und bringt den Ballon in seine, die ankernde und dichtende Funktion ausübende Konfiguration. Die jeweiligen physiologisch wirkenden Kräfte werden vom Katheterballon aufgenommen und so eine weitestgehend druckneutrale ano-rektale Ballonplatzierung ermöglicht, was die Wahrscheinlichkeit ungewollter und verfrühter Triggerwirkungen weitgehend ausschließt.

Eine volumenkontrollierte Füllung des Ballons kann ebenfalls zwei-schrittig erfolgen, wobei initial eine inkomplette Befüllung erfolgt, und der Ballon im zweiten Füllschritt mit einem triggernd wirkenden Volumen befüllt wird. Die sich bei den jeweiligen Volumina im trans-analen Ballon einstellenden Drucke sollen dabei bevorzugt in die in Fig. 6 beschriebenen Druckbereiche (29,30) fallen, und sind vom Anwender, wie bei der druckkontrollierten Befüllung, für sich individuell zu ermitteln.

Zur Begrenzung des Fülldrucks bzw. Vermeidung kritisch hoher Ballonfülldrucke kann sowohl bei manometer- als auch spritzenbetätigter Füllung ein Druckbegrenzungsventil 31 zwischen Füllelement und Katheter eingefügt werden, welches beispielsweise Ballonfülldrucke über 120 mbar vermeidet.

Fig. 7 zeigt eine bevorzugte Ausführungsform eines Einlaufkatheters, der am proximalen Ende in zwei, vorzugsweise fest mit dem Schaft verbundene Zuleitungen übergeht, wobei die Zuleitung 32 mit einem vorzugsweise beutelartigen Behälter 33 mit Einlaufmedium fest verbunden ist, und somit eine für die Anwendung gebrauchsfertige Einheit von Katheter und Medium darstellt.

Das Volumen des Behälters 33 ist dabei so bemessen, dass dieser ca. 80 bis 120 ml Irrigationslösung aufnimmt. Die relativ kleine Menge wird vom Anwender manuell ausgepresst und so, durch wiederholtes Pressen in das Rektum eingebracht. Um ein Greifen des Behälters auch bei eingeschränkter Handmotorik zu ermöglichen, ist der Behälter vorzugsweise zylindrisch, mit einem Durchmesser von ca. 4-6 cm aus geformt.

Die Verbindung 32 ist vorzugsweise mit einem durch Knickung zerbrechbaren Siegel 35 ausgestattet sein, welches die Irrigationslösung frei gibt. Ebenfalls vorteilhaft ist ein im flüssigkeitsführenden Schenkel der anwendungsfertigen Vorrichtung integriertes Ruckschlagventil 36, welches die gerichtete Entleerung des Behälters ohne Rückstrom ermöglicht.

Eine weitere Zuleitung 34 kann direkt mit einem Pumpmanometer 27 oder einer Füllspritze verbunden werden.

Das Ballonelement 2 besteht aus einer dünnwandigen Weichfolie im Wandstärkenbereich von 5 bis 100 Mikrometern. Vorteilhaft sind Folien im Stärkenbereich von 5 bis 40 Mikrometern. Besonders bevorzugt sind wiederum Wandstärken von 5 bis 15 Mikrometern.

Die Verwendung von nur gering volumen-dehnbarer Materialien wie z.B. Polyurethan (PUR), beispielsweise der Spezifikation Pellethane 2363 80A bis 90A, Dow Chemical Corp., wird bevorzugt, da diese Materialien auch bei Ballonfolien im niedrigsten Wandstärkenbereich im Druckbereich von ca. 10 bis 120 mbar eine gute Formstabilität aufweisen.

Solche dünnwandigen, in komplexer Form ausgeformte PUR-Ballonfolien können bevorzugt durch Blasformung ("hot-molding") aus zuvor extrudiertem Rohschlauchmaterial hergestellt werden, was, bei entsprechender Streckung des Schlauchrohlings vor der Temperierung, eine polymere Orientierung ermöglicht, und den geformten Ballonfolien eine außerordentliche mechanische Festigkeit verleiht.

Denkbar ist ebenfalls die Verwendung von Polyurethanen niedriger Shore Härten, wie z.B. des Bereichs von 60 bis 75A, die den Katheterballon im Wandstärkenbereich von unter 40 $\mu$m, und bevorzugt unter 15$\mu$m, im anwendungstypischen Fülldruckbereich von 10 bis 120 $\mu$m, bei relativem Verlust an Formstabilität, mit einem volumendehnbaren Verhalten ausstatten.

Alternativ können beispielsweise auch nicht-volumendehnbare Materialien, wie Polyethylene, PVC oder Mischungen der genannten Materialien mit Polyurethan verwendet werden.

Erfindungsgemäße Ballonfolien können auch direkt aus der extrudierten, noch weichen, weitgehend amorphen Schlauchmasse geformt werden (in-line molding), wobei die erreichbaren Festigkeiten der Folien deutlich kleiner als bei vorextrudierten Schläuchen sind bzw. die erreichbaren Wandstärken deutlich höher als bei der Formung aus vorextrudiertem Material sind.

Denkbar für die Herstellung sind auch Tauchverfahren bei Verwendung flüssiger PVC oder PUR Materialien.

Auch das Verschweißen von singulären Folienlagen zu Ballonkörpern ist vorstellbar.

Die Verbindung des Ballons mit dem Schaftkörper erfolgt durch Verklebung, durch thermische Verfahren oder alternativ auch durch Aufschrumpfen der Ballonenden auf den Schaftkörper.

Der intra-rektale Ballonanteil 4 soll im frei entfalteten, nicht mit Druck beaufschlagten Zustand, bevorzugt einen Durchmesser von ca. 30-60 mm haben, im mittigen Taillenbereich 3 etwa 10 bis 30 mm aufweisen und im prä-analen Bereich 5 etwa 30-50 mm. Das mittige Segment 3 soll ca. 20-40 mm Länge aufweisen, die endständigen Segmente 4 und 5 jeweils eine Länge von ca. 20-40 mm.

Ist der Katheterballon in besonderer Weise zur sequentiellen Befüllung, mit einer optionalen intra-rektalen Ballontriggerung vorgesehen, hat der intra-rektale Ballonanteil 4 im frei entfalteten, nicht mit Druck beaufschlagten Zustand, bevorzugt einen Durchmesser von ca. 40-80 mm und weist eine Länge von bevorzugt 30-60 mm auf.

Neben der trans-analen Anwendung des Einlaufkatheters, können die erfindungsgemäßen Ausführungen auch für die perforationssichere Platzierung eines trans-anal eingelegten Drainagerohres unter anderem auch für die dauerhafte Ableitung von Stuhl aus dem Darm eines Patienten verwendet werden. Ferner ist eine Anwendung der beschriebenen Kathetertechnik in chirurgisch angelegten Stomata/Öffnungen oder weiteren natürlichen Körperöffnungen denkbar.

**Patentansprüche**

1. Vorrichtung zum trans-analen Einbringen eines Einlaufs in das Rektum bzw. Colon eines Patienten, umfassend einen aufblasbaren Ballon (2) von durch Präformierung bei der Herstellung vorgegebener, taillierter, insbesondere hantel- oder sanduhrförmiger Gestalt, mit zwei endständigen Ballonabschnitten (4,5) von größerem Radius ($D_1$/2; $D_2$/2) und etwa sphärischer oder diskoider Gestalt, sowie einem dazwischen angeordneten, mittigen, verjüngten Ballonabschnitt (3) von verringertem Radius, welcher trans-anal platziert wird, derart, dass der distal anschließende, radial erweiterte Ballonabschnitt (4) intra-rektal platziert ist und der proximal anschließende, radial erweiterte Ballonabschnitt (5) extrakorporal, **dadurch gekennzeichnet, dass** die beiden Ballonenden (6,7) sich auf das Schaftmaß eines den Ballon (2) tragenden Katheterschaftes (1) verjüngen und ab einem Wendepunkt (WP) ihres Längsschnittes jeweils einfach nach innen umgestülpt und/oder invertiert, d.h. um einen bestimmten Betrag (B) in das Balloninnere eingestülpt, sind und in diesem einfach invertierten oder umgestülpten Zustand auf der vorzugsweise äußeren Mantelfläche des Katheterschaftes (1) fixiert sind, derart, dass sich bei Befüllung des Ballons (2) die beiden radial erweiterten Ballonabschnitte (4,5) in entgegengesetzten, axialen Richtungen aufeinander zu bewegen, und wobei die beiden radial erweiterten Ballonabschnitte (4,5) gegenüber dem verjüngten, mittigen Ballonanteil (3) derart vergrößert sind, dass beim trans-anal platzierten Ballon (2) die beiden radial erweiterten Ballonabschnitte (4,5) im Laufe der Befüllung sich über den mittigen, verjüngten Ballonabschnitt (3) stülpen und sich gegenläufig auf den Anus zu bewegen und sich so dem jeweiligen analen Situs dichtend anschmiegen und dadurch ihre Relativbewegung begrenzen, wodurch in situ die distale Spitze des Katheterschaftes (1) in dessen nicht ausgelenktem Ruhezustand in eine geschützte, Verletzungen vermeidende Position innerhalb des intra-rektalen Ballonabschnitts (4) retrahiert und dabei vollständig proximal eines distalen Scheitelpunktes (8) eines Kreises (9) um einen Mittelpunkt (M) auf der Symmetrieachse (X) zu liegen kommt, dessen Tangente in dem Wendepunkt (WP) der dortigen Tangente an den nicht umgestülpten Längsschnitt des Ballons (2) entspricht.

2. Vorrichtung nach Anspruch 1, **dadurch gekennzeichnet, dass** die distale Spitze des Katheterschaftes (1) in dessen nicht ausgelenktem Ruhezustand vollständig proximal einer von der Symmetrieachse (X) lotrecht durchsetzten Ebene (Z) zu liegen kommt, welche den intra-rektalen Ballonabschnitt (4) vollständig von distal tangiert.

3. Vorrichtung nach Anspruch 1 oder 2, **dadurch gekennzeichnet, dass** der mittige, verjüngte Ballonabschnitt (3) von den etwa sphärischen oder diskoiden Ballonabschnitten (4,5) durch je einen Wendepunkt des Querschnittsradius getrennt ist und im dehnungsfrei befüllten Zustand eine axiale Länge (A) aufweist, die vorzugsweise größer ist als sein minimaler Durchmesser.

4. Vorrichtung nach einem der Ansprüche 1 bis 3, **dadurch gekennzeichnet, dass** die umgestülpten oder invertierten Ballonenden (6,7) im dehnungsfrei befüllten Zustand jeweils eine axiale Erstreckung $(B_1,B_2)$ aufweisen, die vorzugsweise größer ist als ihr minimaler Durchmesser.

5. Vorrichtung nach einem der vorhergehenden Ansprüche, **dadurch gekennzeichnet, dass** die Summe der axialen Erstreckungen $(B_1,B_2)$ der beidseitigen Inversionen der Ballonenden (6,7) im dehnungsfrei befüllten Zustand mindestens der Länge (A) des mittigen, verjüngten Ballonabschnitts (3) entspricht:

$$B_1 + B_2 \geq A.$$

6. Vorrichtung nach einem der vorhergehenden Ansprüche, wobei die Katheterspitze um ein Maß (C) über die vordere Fixierungslinie (11) des vorderen Ballonendes (6) an dem Katheterschaft (1) hinaus ragt, vorzugsweise **dadurch gekennzeichnet, dass** die Inversionstiefe (B) größer oder gleich der halben Länge (A) plus der Länge (C) des prominenten Spitzenstückes (18) ist:

$$B_1 = B_2 \geq A/2 + C,$$

oder alternativ, weniger bevorzugt:

$$B_1 = B_2 \geq A/2 + C/2.$$

7. Vorrichtung nach einem der vorhergehenden Ansprüche, **dadurch gekennzeichnet, dass** die Katheterspitze sich auch bei darmwärts gerichteter axialer Auslenkung des Katheterschaftes (1), bei anwendungstypisch wirkenden Kräften, nicht über eine Distanz (W) über den Scheitelpunkt (8) des vorderen Ballonradius hinaus bewegt und sich die Distanz (W) aus der Distanz des Scheitelpunktes (8) und dem Scheitelpunkt (20) ergibt, wobei der Scheitelpunkt (20) definiert ist als der vordere Schnittpunkt der Symmetrieachse (X) mit einem Kreis vom Durchmesser (D) um einen Mittelpunkt auf der Symmetrieachse (X) auf Höhe der Befestigung des intra-rektalen Ballonabschnittes (4) an dem Katheterschaft (1).

8. Vorrichtung nach einem der vorhergehenden Ansprüche, **dadurch gekennzeichnet, dass** die Hülle des evakuierten, anwendungsbereiten Ballons (2) derart auf dem Katheterschaft (1) platziert bzw. dicht anliegend an diesen geschmiegt ist, dass der intrarektale und trans-anale Ballonanteil (3) im Schaftbereich zwischen den Fixierungen des oberen und unteren Ballonendes (6,7) liegen, und sich der prä-anale Ballonanteil (3) nach proximal taschenartig über die Griffmulden (12) erstreckt.

9. Vorrichtung nach einem der vorhergehenden Ansprüche, **dadurch gekennzeichnet, dass** die der Katheterschaft (1) im Bereich seiner trans-analen Platzierung innerhalb des Analkanals eine Taillierung (23) aufweist

10. Vorrichtung nach einem der vorhergehenden Ansprüche, **dadurch gekennzeichnet, dass** im Kanal (25) des Katheterschaftes (1) ein Rückschlagelement eingefügt ist, welches einen retrograden, vom Patienten weg gerichteten Rückstrom von Flüssigkeit durch den Katheter ausschließt, wobei vorzugsweise das Rückschlagelement im Kanal (25) aus einem dünnwandigen Schlauchelement von wenigen Mikrometern Wandstärke, vorzugsweise 5 bis 15 Mikrometern Wandstärke, und von dem Durchmesser des Kanals (25) besteht, wobei das distale Ende des Schlauches über eine Länge von ca. 5 bis 10 mm frei im Kanal (25) liegt, und dessen proximales Ende dicht schließend mir der Innenwandung des Kanals (25) verbunden ist, so dass gewährleistet ist, dass sich das Schlauchelement bei spitzenwärts gerichtetem Durchfluss des Mediums durch den Kanal öffnet, und sich das Schlauchelement bei entgegengesetzt gerichtetem Fluss kollabierend verschließt.

11. Vorrichtung nach einem der vorhergehenden Ansprüche, **dadurch gekennzeichnet, dass** der erfindungsgemäße Einlaufkatheter über eine feste Schlauchverbindung (32) mit einem vorzugsweisen beutelartigen Behälter (33) für die Irrigationsflüssigkeit als anwendungsfertiges Einmalprodukt verbunden ist, wobei die feste Schlauchverbindung (32) bevorzugt mit einem Brechsiegel (35) und/oder mit einem Rückschlagventil (36) ausgestattet ist.

**12.** Vorrichtung nach einem der vorhergehenden Ansprüche mit einem Einmalkatheter, **dadurch gekennzeichnet, dass** der Einmalkatheter über eine Befülleitung (34) mit einer vorzugsweise wiederverwendbaren Befüllvorrichtung (27) verbindbar ist, wobei die Befüllvorrichtung (27) als vorzugsweise manuell bedienbarer Pumpballon mit einem den Ballonfülldruck anzeigenden Manometer ausgeführt ist.

**13.** Vorrichtung nach einem der vorhergehenden Ansprüche, **gekennzeichnet durch** ein den Ballondruck begrenzendes Ventilelement, das in oder an einer wiederverwendbaren Befüllvorrichtung (27) angebracht ist oder in der Ballonfüllleitung (34) des vorzugsweise für Einmalanwendung ausgelegten Katheters bzw. der Katheter-Beutel-Einheit integriert ist.

## Claims

**1.** Apparatus for the transanal instillation of an enema into the rectum respectively colon of a patient, comprising an inflatable balloon (2) of waistline, in particular dumbbell or hourglass-shaped form given by preforming in the manufacturing process with two terminal balloon sections (4,5) of larger radius ($D_1/2$; $D_2/2$) and approximately spherical or discoid shape, as well as a centered tapered balloon section (3) of reduced radius arranged in between, which is transanally placed in such a way that the distally adjoining radially extended balloon section (4) is placed intrarectal, and the proximally adjoining radially extended balloon section (5) extracorporeal, **characterized by the fact** that both of the balloon ends (6,7) are tapering down to the shank dimension of a catheter shank (1) carrying the balloon (2), and from an inflection point (WP) of its longitudinal section each of the same are everted onefold inside and/or inverted, i.e. are pushed into the inside of the balloon by a certain amount (B), and in this onefold inverted or pushed-in condition are preferably fixed on the outer lateral surface of the catheter shank (1) in such a way that during filling the balloon (2), the both radially extended balloon sections (4,5) are approaching each other in opposite axial directions, and where both radially extended balloon sections (4,5) are enlarged relative to the centered tapered down balloon section (3) in such a way that at the transanally placed balloon (2), both radially extended balloon sections (4,5) are folding over the tapered, centered balloon section (3) in the course of filling, and are approaching towards the anus in opposite directions, thus clinging the respective anus situs in a sealing manner and thereby are limiting their relative movement, whereby in situ the distal tip of the catheter shank (1) in its undeflected static state retracts to a protected, injuries avoiding position within the intrarectal balloon section (4), and thereby is positioned completely proximal to a distal vertex (8) of a circle (9) around a center (M) on the symmetry axis (X), where the tangent of which in the inflection point (WP) corresponds to the tangent there at the longitudinal section of the not pushed-in balloon (2).

**2.** Apparatus according to claim 1, **characterized by** the fact that the distal tip of the catheter shank (1) in its undeflected static state is positioned completely proximal to plane (Z) being perpendicularly penetrated by the symmetry axis (X), which is completely tangent to the intrarectal balloon section.

**3.** Apparatus according to claim 1 or 2, **characterized by** the fact that the centered tapered down balloon section (3) is separated from the approximately spherical or discoidal balloon sections (4,5) by one each inflection point of the cross-sectional radius, and in its unexpanded filled condition exhibits an axial length (A), which is preferably greater than its minimum diameter.

**4.** Apparatus according to one of the claims 1 through 3, **characterized by** the fact that the pushed-in or inverted balloon ends (6,7) in the unexpanded filled condition each exhibit an axial extent ($B_1, B_2$), which is preferably greater than their minimum diameter.

**5.** Apparatus according to one of the foregoing claims, **characterized by** the fact that the sum of the axial extents ($B_1, B_2$) of the two-sided inversions of the balloon ends (6,7) in the unexpanded filled condition at least corresponds to the length (A) of the centered tapered down balloon section (3):

$$B_1 + B_2 \geq A.$$

**6.** Apparatus according to one of the foregoing claims, where the catheter tip protrudes by a measure (C) beyond the front fixing line (11) of the front balloon end (6) at the catheter shank (1), preferably **characterized by** the fact that the inversion depth (B) is greater than or equal to half of the length (A) plus the length (C) of the prominent tip piece (18):

$$B_1 = B_2 \geq A/2 + C,$$

or alternatively, less preferred:

$$B_1 = B_2 \geq A/2 + C/2.$$

7. Apparatus according to one of the foregoing claims, **characterized by** the fact that the catheter tip also at intestinally directed axial deflection of the catheter shank under application-typically acting forces does not move more than a distance (W) beyond the vertex (8) of the frontal balloon radius, and where such distance (W) results from the distance between vertex (8) and vertex (20), and where the vertex (20) is defined as the frontal point of intersection of the symmetry axis (X) with a circle of diameter (D) around a center point on the symmetry axis (X) at the height of the attachment of the intrarectal balloon section (4) to the catheter shank (1).

8. Apparatus according to one of the foregoing claims, **characterized by** the fact that the envelope of the evacuated, ready-to-use balloon (2) is placed on the catheter shank (1) in such a way respectively is tightly fitted snug to the same so that the intrarectal and transanal balloon portion is located in the shank area between the fixations of the upper and the lower balloon ends (6,7), and the pre-anal balloon portion extends to proximal pocket-like over the grasping recesses (12).

9. Apparatus according to one of the foregoing claims, **characterized by** the fact that the catheter shank (1) exhibits a waistline shape (23) in the area of its transanal placement within the anal channel.

10. Apparatus according to one of the foregoing claims, **characterized by** the fact that a non-return valve type element is integrated in the channel (25) of the catheter shank (1) that excludes any retrograde, away from the patient directed return flow of liquid through the catheter, at which such non-return element in the channel (25) preferably consists of a thin-wall hose element of a few micrometers ($\mu$m) wall thickness, preferably of 5 to 15 micrometers ($\mu$m) wall thickness, and of the diameter of the channel (25), where the distal end of the hose lies freely inside the channel (25) over a length of ca. 5 to 10 mm, and where its proximal end is connected tightly closing up with the internal wall of the channel (25) in order to ensure that the hose element opens when the flow of medium through the channel is directed towards the tip, and the hose element closes up by collapsing when flow occurs in the opposite direction.

11. Apparatus according to one of the foregoing claims, **characterized by** the fact that the enema catheter according to the invention is connected to a preferably bag-like receptacle (33) for the irrigation liquid via a fixed hose connection (32) in form of a single-use product ready for application, and where the fixed hose connection (32) is preferably provided with a breakable seal (35) and/or with a non-return valve (36).

12. Apparatus according to one of the foregoing claims, **characterized by** the fact that the enema catheter according to the invention is connectable to a preferably reusable filling device (27) via a filling line (34), where the filling device (27) is executed as a preferably manually operable pumping balloon with a manometer indicating the filling pressure inside the balloon.

13. Apparatus according to one of the foregoing claims, **characterized by** a valve element limiting the balloon pressure that is attached inside or at a reusable filling device (27), or is integrated into the balloon filling line (34) of the catheter respectively catheter-bag unit preferably designed for single-use application.

**Revendications**

1. Dispositif pour l'introduction transanale d'un lavement dans le rectum ou le côlon d'un patient, comprenant un ballonnet (2) gonflable auquel a été conférée une forme cintrée prédéterminée, en particulier en haltère ou en sablier, par préfaçonnage lors de la fabrication, ce ballonnet (2) présentant deux sections terminales (4,5) de plus grand rayon ($D_1/2$ ; $D_2/2$) et de forme approximativement sphérique ou discoïde, ainsi qu'une section (3) centrale, amincie, interposée de plus petit rayon, laquelle est placée de manière transanale de sorte que la section (4) distalement adjacente et radialement étendue du ballonnet (2) soit placée à l'intérieur du rectum et que la section (5) proximalement adjacente et radialement étendue du ballonnet (2) soit placée à l'extérieur du corps, **caractérisé en ce que** les deux extrémités (6,7) du ballonnet (2) s'amincissent jusqu'à la dimension d'une tige de cathéter (1) portant le ballonnet (2) et à partir d'un point d'inflexion (WP) de leur section longitudinale sont respectivement facilement retournables à l'intérieur et/ou inversées, c'est-à-dire retournées d'une valeur bien définie (B) à l'intérieur du ballonnet et sont fixées à l'intérieur de ce dernier dans cet état facilement inversé ou retournable de préférence sur la surface périphérique extérieure de la tige de cathéter (1) de sorte que, lorsque que le ballonnet (2) est rempli, les deux sections (4,5) radialement étendues du ballonnet (2) se déplacent l'une vers l'autre dans des directions axiales opposées, et **en ce que** les deux sections (4,5) radialement étendues du ballonnet (2) sont élargies par rapport à la section (3) centrale amincie du ballonnet (2) de sorte que, lorsque le ballonnet (2) est placé de façon transanale, les deux sections (4,5) radialement étendues du ballonnet (2) se retournent sur la section (3) centrale amincie du ballonet (2) lors du remplissage, et qui se déplacent contrairement en direction de l'anus et qui se blottent ainsi contre le situs anal correspondant d'une manière colmatante et limitent ainsi leur mouvement relatif ce qui rétracte l'extrémité distale de la tige de cathéter (1) à son état de repos non dévié dans une position protégée, évitant les blessures à l'intérieur de la section intrarectale (4) du ballonnet (2) et dans ce contexte se trouve complètement proximalement d'un sommet distal (8) d'un cercle (9) autour d'un centre (M) sur l'axe de symétrie (X) dont la tangente au point d'inflexion (WP) de la tangente s'y trouvant correspond à la section longitudinale non retournée du ballonnet (2).

2. Dispositif selon la revendication 1, **caractérisé en ce que** l'extrémité distale de la tige de cathéter (1) à son état de repos non dévié se trouve complètement proximalement d'un plan (Z) traversé perpendiculairement par l'axe de symétrie (X), ledit plan étant distalement complètement tangent à la section de ballonnet placée à l'intérieur du rectum.

3. Dispositif selon la revendication 1 ou 2, **caractérisé en ce que** la section (3) centrale amincie du ballonnet (2) est séparée des sections (4,5) approximativement sphériques ou discoïdes du ballonnet (2) par respectivement un point d'inflexion du rayon transversal et présente à l'état rempli exempt de dilation une longueur axiale (A) qui, de préférence, est supérieure à son diamètre minimal.

4. Dispositif selon l'une des revendications 1 à 3, **caractérisé en ce que** les extrémités (6,7) retournées ou inversées du ballonnet (2) présentent à l'état rempli exempt de dilatation respectivement une extension axiale $(B_1, B_2)$ qui, de préférence, est supérieure à son diamètre minimal.

5. Dispositif selon l'une des revendications précédentes, **caractérisé en ce que** la somme des extensions axiales $(B_1, B_2)$ des inversions sur les deux faces des extrémités (6,7) du ballonnet (2) à l'état rempli exempt de dilatation correspond au moins à la longueur (A) de la section centrale (3), amincie du ballonnet (2)

$$B_1 + B_2 \geq A.$$

6. Dispositif selon l'une des revendications précédentes, en ce que l'extrémité de cathéter dépasse d'une dimension (C) de la ligne de fixation avant (11) de l'extrémité (6) avant du ballonnet (2) sur la tige du cathéter (1), de préférence **caractérisé en ce que** la profondeur d'inversion (B) est supérieure ou égale à la demi-longueur (A) plus à la longueur (C) de la pièce d'extrémité proéminente (18) :

$$B_1 = B_2 \geq A/2 + C,$$

ou alternativement, moins préférentiellement :

$$B_1 = B_2 \geq A/2 + C/2.$$

7. Dispositif selon l'une des revendications précédentes, **caractérisé en ce que** l'extrémité du cathéter même lors d'une déviation axiale dirigée vers l'intestin de la tige de cathéter, lors de forces agissant de manière caractéristique à l'application, ne se déplace pas sur une distance (W) au-dessus du sommet (8) du rayon avant du ballonnet et **en ce que** la distance (W) résulte de la distance du sommet (8) et du sommet (20), **en ce que** le sommet (20) est défini comme le point d'intersection avant de l'axe de symétrie (X) avec un cercle de diamètre (D) autour d'un centre sur l'axe de symétrie (X) à hauteur de la fixation de la partie intrarectale (4) du ballonnet (2) sur la tige de cathéter (1).

8. Dispositif selon l'une des revendications précédentes, **caractérisé en ce que** l'enveloppe du ballonnet (2) mis au vide, prêt à l'utilisation, est placée sur la tige cathéter (1) ou épouse hermétiquement cette dernière de telle sorte que la section de ballonnet intrarectale ou transanale se trouve dans la zone de tige entre les fixations de l'extrémité supérieure et inférieure du ballonnet (6, 7), et la section de ballonnet pré-anale s'étend proximalement en forme de poche au-dessus des poignées concaves (12).

9. Dispositif selon l'une des revendications précédentes, **caractérisé en ce que** la tige de cathéter (1) dans la zone de son emplacement transanal dans le canal anal présente un cintrage (23).

10. Dispositif selon l'une des revendications précédentes, **caractérisé en ce qu'**un élément anti-retour est inséré dans le canal (25) de la tige de catheter (1), ledit élément anti-retour excluant un retour de liquide rétrograde, éloigné du patient, par le cathéter, **en ce que** de préférence l'élément anti-retour dans le canal (25) est constitué d'un élément de tuyau à paroi mince d'une épaisseur de paroi de quelques micromètres, de préférence d'une épaisseur de paroi de 5 à 15 micromètres, et du diamètre du canal (25), **en ce que** l'extrémité distale du tuyau s'étend sur une longueur comprise approximativement entre 5 et 10 mm librement dans le canal (25), et dont l'extrémité proximale est reliée de manière étanche à la paroi intérieure du canal (25) ce qui garantit que l'élément de tuyau s'ouvre à écoulement du fluide dirigé vers l'extrémité par le canal, et que l'élément de tuyau se ferme en s'affaissant à flux dirigé en sens opposé.

11. Dispositif selon l'une des revendications précédentes, **caractérisé en ce que** le cathéter de lavement selon l'invention est relié par l'intermédiaire d'un raccord pour tuyau fixe (32) à un réservoir (33) de préférence en forme de poche pour le liquide d'irrigation comme produit à usage unique prêt à l'emploi, **en ce que** le raccord pour tuyau fixe (32) est équipé de préférence d'un sceau qui peut être brisé (35) et/ou d'un clapet anti-retour (36).

12. Dispositif selon l'une des revendications précédentes avec un cathéter à usage unique, **caractérisé en ce que** le cathéter à usage unique peut être relié par l'intermédiaire d'une conduite de remplissage (34) à un dispositif de remplissage (27) de préférence réutilisable, **en ce que** le dispositif de remplissage est conçu de préférence comme ballonnet de pompage à commande manuelle avec un manomètre indiquant la pression de remplissage du ballonnet.

13. Dispositif selon l'une des revendications précédentes, **caractérisé par** un élément limiteur de pression du ballonnet qui est monté dans ou sur un dispositif de remplissage réutilisable (27) ou qui est intégré dans la conduite de remplissage du ballonnet (34) du cathéter conçu de préférence à usage unique ou bien dans l'unité cathéter poche.

## Fig.1a

Fig.1b

Fig.1e

Fig.1c

Fig.1d

# Fig.2

Fig.3

Fig.4

Fig.5

Fig.6

Fig.7

**EP 2 744 445 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2008103788 A1
- DE 102004033425 B4
- WO 2007118621 A1